# EUROPEAN PATENT APPLICATION

(11) **EP 4 699 612 A1**
(43) Date of publication of application: **25.02.2026**
(21) Application number: 24792755.1
(22) Date of filing: 19.04.2024
(51) Int. Cl.: A61K 35/747, A23L 33/135, A61K 8/99, A61P 1/02, A61P 1/04, A61P 9/10, A61P 31/04, A61P 35/00, A61Q 11/00

(54) **PROLIFERATION INHIBITOR FOR PERIODONTAL DISEASE BACTERIUM AND APPLICATION FOR SAME**

(30) Priority: 19.04.2023 JP 2023068906
(71) Applicant: Asfreya Inc., Tokyo 136-0082 (JP)
(72) Inventor: OCHIYA Takahiro, Tokyo 104-0053 (JP)
(74) Representative: J A Kemp LLP
(86) International application number: PCT/JP2024/015542
(87) International publication number: WO 2024/219483

(57) **Abstract**

An object is to provide a novel active ingredient to inhibit the growth of a periodontal pathogen.

[Solution] The periodontal pathogen growth inhibitor of the present invention is characterized by including at least one of membrane vesicles of Lactobacillus reuteri and membrane vesicles of Lactobacillus casei. The method for inhibiting the growth of a periodontal pathogen of the present invention is characterized in that the periodontal pathogen growth inhibitor of the present invention and a periodontal pathogen are allowed to coexist. The periodontal pathogen is, for example, bacteria of the genus Fusobacterium, bacteria of the genus Porphyromonas and bacteria of the genus Tannerella.

## Description

### [TECHNICAL FIELD]

The present invention relates to a periodontal pathogen growth inhibitor and its uses.

### [BACKGROUND ART]

Bacteria of the genus Fusobacterium, bacteria of the genus Porphyromonas, bacteria of the genus Tannerella, for example, are oral resident bacteria and known to be involved in periodontal disease. In recent years, the bacteria of the genus Fusobacterium have been further reported to be involved in colorectal cancer. Therefore, inhibiting the growth of periodontal pathogens existing in living bodies is considered to lead to the prevention and treatment of diseases in which the pathogens are involved.

### [SUMMARY OF INVENTION]

### [TECHNICAL PROBLEM]

Therefore, an object of the present invention is to provide a novel active ingredient to inhibit the growth of a periodontal pathogen.

### [SOLUTION TO PROBLEM]

In order to achieve the object, a periodontal pathogen growth inhibitor of the present invention is characterized by including at least one of membrane vesicles of Lactobacillus reuteri and membrane vesicles of Lactobacillus casei.

A method for inhibiting the growth of a periodontal pathogen of the present invention is characterized in that the periodontal pathogen growth inhibitor of the present invention and a periodontal pathogen are allowed to coexist.

A drug for a periodontal pathogen-related disease of the present invention is characterized by including the periodontal pathogen growth inhibitor of the present invention as an active ingredient.

### [ADVANTAGEOUS EFFECT OF INVENTION]

The present inventors found that membrane vesicles of Lactobacillus reuteri and Lactobacillus casei inhibited the growth of a periodontal pathogen such as bacteria of the genus Fusobacterium, thereby establishing the present invention. The present invention can inhibit the growth of periodontal pathogens and thus, for example, can improve diseases caused by the periodontal pathogens by inhibiting the growth thereof.

### [BRIEF DESCRIPTION OF DRAWING]

Fig. 1 is a graph showing the results of particle diameter distribution of membrane vesicles derived from Lactobacillus reuteri prepared in Example 1;
Fig. 2 is a graph showing the results of particle diameter distribution of membrane vesicles derived from Lactobacillus casei prepared in Example 1; and
Fig. 3 is photographs showing the culture state of bacteria of the genus Fusobacterium cultured in the presence of membrane vesicles derived from Lactobacillus reuteri or Lactobacillus casei in Example 2.

### [DESCRIPTION OF EMBODIMENT]

[1] A periodontal pathogen growth inhibitor, characterized by including at least one of membrane vesicles of Lactobacillus reuteri and membrane vesicles of Lactobacillus casei.
[2] The growth inhibitor according to [1], wherein the periodontal pathogen is at least one selected from the group consisting of bacteria of the genus Fusobacterium, bacteria of the genus Porphyromonas and bacteria of the genus Tannerella.
[3] The growth inhibitor according to [2], wherein the bacteria of the genus Fusobacterium are Fusobacterium nucleatum.
[4] The growth inhibitor according to [2], wherein the bacteria of the genus Porphyromonas are Porphyromonas gingivalis.
[5] The growth inhibitor according to [2], wherein the bacteria of the genus Tannerella are Tannerella forsythensis.
[6] The growth inhibitor according to any one of [1] to [5], wherein the membrane vesicles are membrane vesicles isolated from cultures of Lactobacillus reuteri.
[7] The growth inhibitor according to any one of [1] to [5], wherein the membrane vesicles are membrane vesicles isolated from cultures of Lactobacillus casei.
[8] A method for inhibiting the growth of a periodontal pathogen, characterized in that the periodontal pathogen growth inhibitor according to any one of [1] to [7] and a periodontal pathogen are allowed to coexist.
[9] The growth inhibition method according to [8], wherein the coexistence is coexistence in vivo or in vitro.
[10] The growth inhibition method according to [8] or [9], wherein the coexistence is coexistence in a living body of a human or a non-human animal.
[11] A drug for a periodontal pathogen-related disease, characterized by including the periodontal pathogen growth inhibitor according to any one of [1] to [7] as an active ingredient.
[12] The drug for a periodontal pathogen-related disease according to [11], further including an excipient.
[13] The drug for a periodontal pathogen-related disease according to [11] or [12], wherein the periodontal pathogen-related disease is myocardial infarction, cerebral infarction, an oral disease or a gastrointestinal disease.
[14] The drug for a periodontal pathogen-related disease according to [13], wherein the oral disease is periodontal disease or tooth decay.
[15] The drug for a periodontal pathogen-related disease according to [13], wherein the gastrointestinal disease is large bowel disease.
[16] The drug for a periodontal pathogen-related disease according to [15], wherein the large bowel disease is colitis or colorectal cancer.
[17] Membrane vesicles of Lactobacillus reuteri or membrane vesicles of Lactobacillus casei for use in the inhibition of the growth of a periodontal pathogen or a drug for a periodontal pathogen-related disease.
[18] Membrane vesicles of Lactobacillus reuteri or membrane vesicles of Lactobacillus casei for use in the production of a periodontal pathogen growth inhibitor or a drug for a periodontal pathogen-related disease.

Unless otherwise mentioned, terms used in this description have meanings commonly used in the art.

In this description, "membrane vesicle" is a bag-shaped body released from the biomembrane of bacteria and also called membrane vesicle (MV).

In this description, "growth inhibition" means, for example, that the degree of the growth of target bacteria in the presence of an active ingredient is low compared to the degree of the growth of the same target bacteria in the absence of the active ingredient. In addition, the "growth inhibition" may mean, for example, that target bacteria die out by contact with an active ingredient.

In this description, "treatment" may have, for example, any meaning of the inhibition of aggravation, relief (improvement), remission and cure (complete recovery) of symptoms in a target disease. In this description, the "treatment" may also include, for example, "prevention" in its broad sense. In this description, the "prevention" includes, for example, meanings of inhibiting the onset of a target disease, delaying the onset and the like. In this description, for example when the "treatment" has a narrow sense, the "treatment" in this description can be read, for example, as prevention and the present invention may include a treatment method and prevention method.

The present invention will now be described by way of specific examples thereof. It should be noted, however, that the present invention is not restricted to these examples. In addition, examples in the respective inventions can be used to each other.

### (1) Growth inhibitor

As described above, the periodontal pathogen growth inhibitor of the present invention is characterized by including at least one of membrane vesicles of Lactobacillus reuteri and membrane vesicles of Lactobacillus casei. The periodontal pathogen growth inhibitor of the present invention is hereinafter also referred to as "growth inhibitor."

Bacteria targeted by the growth inhibitor of the present invention are periodontal pathogens. Specific examples of the periodontal pathogens are e.g. bacteria of the genus Fusobacterium, bacteria of the genus Porphyromonas, bacteria of the genus Tannerella and the like, and these are anaerobic gram-negative bacteria and oral resident bacteria. Specific examples of the bacteria of the genus Fusobacterium can include F. nucleatum, F. necrophorum, F. varium, F. mortiferum and the like. Specific examples of the bacteria of the genus Porphyromonas can include e.g. Porphyromonas gingivalis and the like. Specific examples of the bacteria of the genus Tannerella can include e.g. Tannerella forsythensis and the like. The periodontal pathogens targeted by the present invention are not restricted to these examples and examples thereof can include bacteria of the genus Treponema such as Treponema denticola and the like.

The growth inhibitor of the present invention includes at least one of membrane vesicles of Lactobacillus reuteri and membrane vesicles of Lactobacillus casei as an active ingredient and may include, for example, only membrane vesicles of Lactobacillus reuteri of the two or only membrane vesicles of Lactobacillus casei of the two. The growth inhibitor of the present invention may further include, for example, as membrane vesicles, those other than membrane vesicles of Lactobacillus reuteri and membrane vesicles of Lactobacillus casei.

In the growth inhibitor of the present invention, the amount of the membrane vesicles contained is not particularly restricted and can be appropriately set depending on, for example, the intended use. Specific examples thereof are described below.

Lactobacillus reuteri is an anaerobic gram-positive lactobacillus, classified into the genus Bacillus and having Lactobacillus reuteri as the scientific name thereof. The strain of Lactobacillus reuteri in the present invention is not particularly restricted and examples thereof can include BAA-2837, DSM 17938, ATCC PTA 6475 and the like.

Lactobacillus casei is an anaerobic gram-positive lactobacillus, classified into the genus Bacillus and having Lactobacillus casei as the scientific name thereof. The strain of Lactobacillus casei in the present invention is not particularly restricted and examples thereof can include K-1, Shirota, BC-90, SBR1202, NY1301 and the like.

Lactobacillus reuteri and Lactobacillus casei are gram-positive bacteria as described above and membrane vesicles derived from these bacterial cells can be said to be, for example, bag-shaped bodies formed from the lipid bilayer, a biomembrane, specifically a spherical structure.

The membrane vesicles of Lactobacillus reuteri and the membrane vesicles of Lactobacillus casei can be produced, for example, by culturing at least one of Lactobacillus reuteri and Lactobacillus casei as source bacteria. A method for culturing the source bacteria is provided below as an example, and in the following example, the source bacteria may be, for example, both Lactobacillus reuteri and Lactobacillus casei, only Lactobacillus reuteri or only Lactobacillus casei.

Culturing can be performed by seeding the source bacteria in a medium and incubating the bacteria. Membrane vesicles secreted from the source bacteria are released to the medium by culturing the source bacteria. The culture conditions are not particularly restricted and common culture conditions for lactic acid bacteria (preferably bacteria of the genus Lactobacillus) can be selected.

The medium is not particularly restricted and examples thereof can include de Man-Rogosa-Sharpe (MRS) medium, arginine containing MRS medium, brain heart infusion (BHI) medium, L-glutamine containing advanced RPMI1640 (final concentration of L-glutamine 10 mM) medium and the like. From the viewpoint of recovering the membrane vesicles, the medium is preferably a liquid medium. The medium may include additives such as amino acids, glucose and the like. For the additives, for example, it is possible to refer to the types and concentrations of amino acids and glucose contained in RPMI1640 medium and DMEM medium and the like.

The culture temperature is not particularly restricted and is, for example, the optimal temperature of lactic acid bacteria (preferably bacteria of the genus Lactobacillus). Specific examples thereof are 28 to 40°C, 29 to 38°C, 30 to 37°C and the like. The culturing time is not particularly restricted and may be appropriately decided depending on, for example, the culture scale, the types of source bacteria and the like. Culturing may be also performed to a confluent state. The optical density (OD) of a culture solution, for example, can be used for judgement of the confluent state and the confluent state can be judged when the value of OD600 reaches a range from about 2.5 to 3.5 as a specific example. Examples of the culturing time can include 12 to 72 hours, 12 to 24 hours, 24 to 48 hours, 48 to 72 hours and the like.

The membrane vesicles can be separated from the culture solution, for example, by removing cultured bacterial cells from the culture solution and recovering the fraction containing the membrane vesicles (e.g. culture supernatant). The separation method is not particularly restricted and examples thereof can include centrifugation of the culture solution and the like. When the cultured bacterial cells and the culture supernatant are separated by centrifugation, the conditions are not particularly restricted and are, for example, 4,000 to 6,000 xg and 10 to 15 minutes.

The present invention can use, for example, the culture solution as the membrane vesicles; however, it is preferred to directly use the fraction obtained by removing cultured bacterial cells from the culture solution (the culture supernatant), use purified material obtained by removing impurities from the culture supernatant, use concentrated material obtained by further concentrating membrane vesicles contained in the culture supernatant, or use purified concentrated material obtained by removing impurities and concentrating membrane vesicles. The impurities can be checked, for example, by a nanoparticle analyzing system or the like.

Examples of the purification method for removing the impurities can include standing, centrifugation such as ultracentrifugation, filtration, chromatography, electrophoresis and the like.

In the case of standing, for example, the culture supernatant is stirred and then left to stand to precipitate impurities and the liquid fraction containing the membrane vesicles is recovered. The standing conditions are not particularly restricted and are, for example, 5 to 60 minutes.

In the centrifugation, for example about the culture supernatant, impurities may be precipitated to recover the liquid fraction containing the membrane vesicles or the membrane vesicles may be precipitated to remove the liquid fraction containing impurities. The latter may be further carried out after the former. When the impurities are precipitated, the centrifugation conditions are not particularly restricted and are, for example, 400 to 3000 xg and 5 to 30 minutes, which is also referred to as coarse centrifugation. In addition, when precipitating the membrane vesicles, for example, ultracentrifugation is preferred and the conditions thereof are not particularly restricted and are, for example, 50,000 to 150,000 xg and 50 to 140 minutes. The precipitation fraction containing the membrane vesicles may be, for example, suspended in a liquid solvent and the suspension liquid may be, for example, stored under refrigeration or freezing.

The filtration is, for example, filtration using a filtering material such as a filter and specific examples thereof include ultrafiltration and the like. As the filtering material, for example, a filtering material through which the membrane vesicles pass or a filtering material through which the membrane vesicles cannot pass may be used. In the former, for example, remaining material on the filtering material is removed as impurities and the liquid fraction (filtrate) containing the membrane vesicles can be recovered. The pore size of the filtering material may be, for example, a size through which the membrane vesicles can pass. In the case of the latter, for example, the filtrate containing impurities may be removed to recover the membrane vesicles captured on the filtering material. The pore size of the filtering material may be, for example, pores through which the membrane vesicles cannot pass. In the latter, the membrane vesicles captured on the filtering material may be, for example, suspended in the liquid solvent.

Examples of the chromatography can include gel filtration chromatography and the like, and examples of the electrophoresis can include free flow electrophoresis, capillary electrophoresis and the like.

The liquid solvent is not particularly restricted and examples thereof can include an aqueous solvent such as water, a buffer solution, saline or a physiological buffer solution.

Examples of the concentration method for concentrating membrane vesicles can include ultracentrifugation, filtration, drying such as lyophilization and the like. In the case of ultracentrifugation, for example, the membrane vesicles are precipitated and the obtained precipitation fraction may be suspended in a desired amount of the liquid solvent. In the case of filtration, for example, the membrane vesicles captured on the filtering material may be suspended in a desired amount of the liquid solvent. In addition, in the case of lyophilization, a liquid containing membrane vesicles (e.g. the culture supernatant, the liquid fraction, the suspension liquid, etc.) is dried and the obtained dried material may be suspended in a desired amount of the liquid solvent.

Separation of the membrane vesicles from the culture solution, purification and concentration can be performed, for example, under cooling conditions to room temperature conditions (e.g. 4 to 37°C) and centrifugation, filtration, chromatography, etc. are preferably performed at 4°C ± 2°C.

The size of membrane vesicles derived from Lactobacillus reuteri used in the present invention can be checked, for example, by particle diameter distribution and the size can be represented, for example, by at least any of mode, mean and SD. The mode can be also said to be, for example, a peak in the particle diameter distribution. The size of membrane vesicles derived from Lactobacillus reuteri described below can be provided as examples.

### (Membrane vesicles derived from Lactobacillus reuteri)

Mode of particle diameter in particle diameter distribution;
Peak 1: e.g. 60 to 80 nm, 71 ± 10, 71 ± 5 (specific example: 71 nm),
Peak 2: e.g. 85 nm to 110 nm, 93 ± 10 nm, 93 ± 5 nm (specific example: 93 nm), and
Peak 3: e.g. 150 to 170 nm, 160 ± 10, 160 ± 5 (specific example: 160 nm).
Mean of particle diameter in particle diameter distribution: e.g. 121 ± 20 nm, 121 ± 10 nm, 121 ± 5 nm (specific example: 121.4 nm).

Membrane vesicles derived from Lactobacillus reuteri may have, for example, one peak, two peaks, three peaks or four or more peaks in the particle diameter distribution. Membrane vesicles derived from Lactobacillus reuteri preferably have, for example, a peak corresponding to any range provided above as examples, and may have any of peak 1, peak 2 and peak 3, may have any two peaks (e.g. peak 1 and peak 2) or may have three peaks as specific examples.

The size of membrane vesicles derived from Lactobacillus casei used in the present invention can be checked, for example, by particle diameter distribution and the size can be represented, for example, by at least any of mode, mean and SD. The mode can be also said to be, for example, a peak in the particle diameter distribution. The size of membrane vesicles derived from Lactobacillus casei described below can be provided as examples.

### (Membrane vesicles derived from Lactobacillus casei)

Mode of particle diameter in particle diameter distribution;
Peak 1: e.g. 90 to 110 nm, 99 ± 10 nm, 99 ± 5 nm (specific example: 99 nm), and
Peak 2: e.g. 120 nm to 140 nm, 136 ± 10 nm, 136 ± 5 nm (specific example: 136 nm).
Mean of particle diameter in particle diameter distribution: e.g. 134 ± 20 nm, 134 ± 10 nm, 134 ± 5 nm (specific example: 134.4 nm).

Membrane vesicles derived from Lactobacillus casei may have, for example, one peak, two peaks or three or more peaks in the particle diameter distribution. Membrane vesicles derived from Lactobacillus casei preferably have, for example, a peak corresponding to any range provided above as examples and may have any of peak 1 and peak 2 or may have both peaks (e.g. peak 1 and peak 2) as a specific example.

The method for measuring the particle diameter distribution of the membrane vesicles is not particularly restricted and a light scattering method such as a laser diffraction method or a dynamic light scattering method, a particle tracking analysis method, a nanoparticle tracking analysis method based on Brownian motion or the like, for example, can be adopted. For the measurement, for example, a commercially available device can be used and in the case of the nanoparticle tracking analysis method as a specific example, NanoSight (trade name, Quantum Design) or the like can be used.

The growth inhibitor of the present invention may include only an active ingredient to inhibit the growth of a periodontal pathogen, or may include the active ingredient and other additive components. In the present invention, the active ingredient is only needed to contain at least one of membrane vesicles of Lactobacillus reuteri and membrane vesicles of Lactobacillus casei as described above, and may be in a state containing membrane vesicles of Lactobacillus reuteri and not containing membrane vesicles of Lactobacillus casei, a state containing membrane vesicles of Lactobacillus casei and not containing membrane vesicles of Lactobacillus reuteri, or a state containing both membrane vesicles of Lactobacillus reuteri and membrane vesicles of Lactobacillus casei. In addition, the present invention may include a component to inhibit the growth of a periodontal pathogen in addition to the membrane vesicles of Lactobacillus reuteri or the membrane vesicles of Lactobacillus casei as the active ingredient.

In the growth inhibitor of the present invention, the other additive components are not particularly restricted and can be appropriately selected depending on, for example, the fields to which the growth inhibitor of the present invention is applied.

Since the membrane vesicles can be prepared, for example, from a culture solution of the source bacteria as described above, the growth inhibitor of the present invention may contain a component other than the membrane vesicles contained in the culture solution. In addition, the fraction containing the membrane vesicles obtained by removing cultured bacterial cells from the culture solution (the culture supernatant) or the fraction obtained by further purifying and/or concentrating the membrane vesicles from the culture supernatant, for example, is preferably used as the membrane vesicles in the growth inhibitor of the present invention as described above. Therefore, it is preferred that the growth inhibitor of the present invention not substantially contain, for example, bacterial cells themselves of Lactobacillus reuteri and Lactobacillus casei as the source bacteria. Not substantially containing bacterial cells means that for example when the growth inhibitor of the present invention is used for a microbiological detection method, viable cells or dead cells of Lactobacillus reuteri and Lactobacillus casei are not more than the detection limit thereof.

The growth inhibitor of the present invention can be used in vivo or in vitro depending on, for example, the purpose.

When the growth inhibitor of the present invention is used in vivo, the growth inhibitor of the present invention may be, for example, a pharmaceutical composition or a food and drink composition.

When the growth inhibitor of the present invention is a pharmaceutical composition, it can be used, for example, to inhibit the growth of a periodontal pathogen in a living body, treat a disease caused by a periodontal pathogen (hereinafter referred to as periodontal pathogen-related disease) and the like. In this case, the pharmaceutical composition of the present invention may be administered, for example, to a target living body. The living body is, for example, a human or a non-human animal and as the non-human animal, for example, mammalian animals such as mice, rats, dogs, cats, monkeys, rabbits, cattle, goats and camels can be provided as examples.

The dosage of the pharmaceutical composition of the present invention is not particularly restricted and it is preferably administered in a pharmaceutically effective amount. The pharmaceutically effective amount can be decided depending on, for example, the administration route, the types of disease, with or without the onset of a disease, severity, age and the like. In addition, the amount of the membrane vesicles contained in the pharmaceutical composition of the present invention is not particularly restricted and is preferably, for example, an amount which is administered in a pharmaceutically effective amount.

The administration route is not particularly restricted and may be, for example, a parenteral or oral route. Examples of the parenteral route can include dermal, subcutaneous, intravenous, intraarterial, intraperitoneal, intranasal, intraoral, inhalation, gastrointestinal such as intestinal and local administration routes and the like.

When the pharmaceutical composition of the present invention is used for parenteral administration, the daily dosage of the membrane vesicles is, for example, 100 to 1,000 mg and the like and the daily frequency of administration is, for example, once to three times, once to twice and once as specific examples. When the pharmaceutical composition of the present invention is used for oral administration, the daily dosage of the membrane vesicles is, for example, 10 to 100 mg and the like and the daily frequency of administration is not particularly restricted and is, for example, once to three times, once to twice and once. The amount of the membrane vesicles contained in the pharmaceutical composition of the present invention is not particularly restricted as described above, and the membrane vesicles can be mixed, for example, to meet the dosages provided as examples.

The pharmaceutical composition of the present invention may include, for example, the active ingredient and other additive components as described above. Examples of the additive components can include pharmaceutically acceptable components and the like. Specific examples thereof can include e.g. an excipient, a carrier (base) and the like. Examples of the excipient and the carrier can include an aqueous solvent such as water, saline or a buffer solution; oil and fat such as soybean oil; petrolatum; an alcohol such as glycerol; a saccharide such as maltose, dextrose and dextrin; a sugar alcohol such as xylitol; a phospholipid; liposome and the like. In addition to these, examples of the additive components can include a binder, a disintegrator, a surfactant, an emulsifier, an antioxidant, a lubricant, a wetting agent, a thickener, a stabilizer, a UV blocking agent, an antiseptic, a preservative, a vitamin, a mineral, a coloring agent and the like.

The dosage form of the pharmaceutical composition of the present invention is not particularly restricted and can be appropriately selected depending on the administration route. Specific examples of the dosage form can include e.g. injections, oral agents (internal medicines), external preparations (external medicines) and the like and examples of the form of the oral agents can include liquid medicines, emulsion agents, gel agents, sol agents, granules, pills, tablets, capsules, tablets and the like. Examples of the external preparations can include transdermal medicines such as dermal medicines, nose drops, eye drops, ear drops, oral medicines, suppositories and the like. Examples of the form of the external preparations can include liquid medicines, emulsion agents, gel agents, sol agents, ointments, granules, pills, tablets, capsules and the like.

The pharmaceutical composition of the present invention may be, for example, a pharmaceutical product, a quasi-drug or a health product.

When the growth inhibitor of the present invention is a food and drink composition, for example, it is a food and drink containing membrane vesicles, the active ingredient. The food and drink composition of the present invention may be, for example, a food or a drink. The food and drink composition of the present invention may be, for example, a health functional food composition.

The intake of the food and drink composition of the present invention is not particularly restricted and the daily intake of the membrane vesicles is, for example, 100 to 1,000 mg and the like and the daily number of intakes is, for example, once to three times, once to twice and once. The amount of the membrane vesicles contained in the food and drink composition of the present invention is not particularly restricted as described above and the membrane vesicles can be mixed, for example, to meet the intakes provided as examples.

The food and drink composition of the present invention may include, for example, the active ingredient and other additive components as described above. Examples of the additive components can include an aqueous solvent such as water, saline or a buffer solution, fat and oil such as soybean oil, an alcohol such as ethanol, a saccharide such as glucose, a sugar alcohol such as xylitol, an emulsifier, an antioxidant, a thickener, an organic acid, a pH adjuster, a stabilizer, an antiseptic, a preservative, a vitamin, a mineral, a flavor, a coloring agent, a filler, a carbonating agent and the like.

### (2) Growth inhibition method

The growth inhibition method of the present invention is characterized in that the periodontal pathogen growth inhibitor of the present invention and a periodontal pathogen are allowed to coexist as described above. For the growth inhibition method of the present invention, the description about the growth inhibitor of the present invention described above can be used. The periodontal pathogen as a target in the present invention is not particularly restricted and examples thereof can include bacteria of the genus Fusobacterium, bacteria of the genus Porphyromonas and bacteria of the genus Tannerella described above and the like.

In the growth inhibition method of the present invention, the step of allowing the growth inhibitor and a periodontal pathogen to coexist (coexistence step) can be said to be, for example, a step of exposing the growth inhibitor to the periodontal pathogen. The step may be, for example, performed in vivo or in vitro depending on the purpose.

When the growth inhibition method of the present invention is performed in vivo, the coexistence step is, for example, a step of administering the growth inhibitor to a target and the target is a living body, i.e. an animal individual. The living body is, for example, a human or a non-human animal as described above.

According to this embodiment, for example, the growth of a periodontal pathogen in a living body can be inhibited. According to this embodiment, for example, diseases related to periodontal pathogens can be also treated by inhibiting the growth of the periodontal pathogens. Examples of periodontal pathogen-related diseases are described below.

Examples of the administration route for the growth inhibitor can include parenteral or oral administration as described above, and the dosage of the growth inhibitor is not particularly restricted and examples described above can be used.

When the growth inhibition method of the present invention is performed in vitro, the coexistence step is, for example, a step of allowing the growth inhibitor and a periodontal pathogen to coexist in a medium. As a specific example, the coexistence step is a step of culturing a periodontal pathogen in the medium containing the growth inhibitor. According to this embodiment, the growth of periodontal pathogens can be inhibited.

The culture conditions for the periodontal pathogens are not particularly restricted. As the type of the medium, for example, ABHK medium, HK agar medium or the like can be used. Examples of the media can include an agar medium and a liquid medium. The culture temperature is not particularly restricted and is, for example, the optimal temperature of a periodontal pathogen. Specific examples thereof are 28 to 40°C, 29 to 38°C and 30 to 37°C. The culturing may be, for example, anaerobic culture or aerobic culture.

The proportion of the growth inhibitor added to the periodontal pathogen is not particularly restricted. As a specific example, the proportion of the growth inhibitor added is, for example, 100 to 1,000 membrane vesicles, the active ingredient, per bacterial cell of the periodontal pathogen. In addition, the concentration of membrane vesicles, the active ingredient, in the medium is, for example, 50 to 100 µg/mL.

### (3) Drug for periodontal pathogen-related disease and treatment method

The drug for a periodontal pathogen-related disease of the present invention (hereinafter referred to as drug for a disease) is characterized by including the periodontal pathogen growth inhibitor of the present invention as the active ingredient. The drug for a disease of the present invention is, for example, a drug for a living body and may be a pharmaceutical product, a quasi-drug or a care product.

For the drug for a disease of the present invention, the description about the growth inhibitor of the present invention in (1) above can be used, and specifically the description about the pharmaceutical composition in (1) above can be used.

The method for treating a periodontal pathogen-related disease of the present invention is characterized by including a step of administering the periodontal pathogen growth inhibitor of the present invention to a patient. In the method for treating a periodontal pathogen-related disease of the present invention, the administration step can be also said to be, for example, a step of administering at least one of membrane vesicles of Lactobacillus reuteri and membrane vesicles of Lactobacillus casei to a patient, in other words.

In the present invention, the periodontal pathogen-related disease is a disease related to a periodontal pathogen. The disease is, for example, myocardial infarction, cerebral infarction, a gastrointestinal disease or an oral disease. The gastrointestinal disease is, for example, large bowel disease and specific examples thereof can include colitis or colorectal cancer. The oral disease is, for example, periodontal disease or tooth decay. These diseases are related to periodontal pathogens. Therefore, the growth of periodontal pathogens is inhibited by administering membrane vesicles, the active ingredient in the growth inhibitor of the present invention and consequently the diseases can be treated.

The administration method for the growth inhibitor is not particularly restricted and may be, for example, parenteral administration or oral administration as described above. In addition, the dosage of the growth inhibitor is not particularly restricted and examples as described above, for example, can be used.

When the target disease is the myocardial infarction, the administration of the growth inhibitor is preferably, for example, oral administration, intravenous administration and the like.

When the target disease is the cerebral infarction, the administration of the growth inhibitor is preferably, for example, oral administration, nasal administration, intravenous administration and the like.

When the target disease is the gastrointestinal disease, the administration of the growth inhibitor is preferably, for example, oral administration, intravenous administration, a suppository and the like.

When the target disease is the oral disease, the administration of the growth inhibitor is preferably, for example, intraoral administration. In the case of intraoral administration, examples of the growth inhibitor can include a mouthwash, an oral ointment, an orally dissolving or disintegrating preparation (e.g. troche or patch) and the like.

### (4) Uses

The present invention is membrane vesicles of Lactobacillus reuteri or membrane vesicles of Lactobacillus casei for use in inhibition of the growth of a periodontal pathogen. The present invention is membrane vesicles of Lactobacillus reuteri or membrane vesicle of Lactobacillus casei for producing the periodontal pathogen growth inhibitor.

The present invention is membrane vesicles of Lactobacillus reuteri or membrane vesicle of Lactobacillus casei for use in the treatment of a periodontal pathogen-related disease. The present invention is membrane vesicles of Lactobacillus reuteri or membrane vesicles of Lactobacillus casei for producing a drug for a periodontal pathogen-related disease.

For the membrane vesicles of Lactobacillus reuteri and membrane vesicles of Lactobacillus casei in the present invention, the description in (1) above can be used.

The present invention will now be described in detail by way of Examples and the like thereof. It should be noted, however, that the present invention is not limited thereto.

### EXAMPLES

### [Example 1]

Respective membrane vesicles were recovered from Lactobacillus reuteri and Lactobacillus casei.

As the source bacteria, Lactobacillus reuteri BAA-2837 strain (ATCC) was used as Lactobacillus reuteri, and Lactobacillus casei YA921 strain separated from a commercially available preparation (generic name: Lactobacillus casei powder, trade name: BIOLACTIS (registered trademark) POWDER) was used as Lactobacillus casei. For culturing the source bacteria, BHI medium (2% yeast extract, 0.2% cysteine) was used and the culture conditions were 37°C, a CO₂ concentration of 5% and an anaerobic state.

Colonies of the source bacteria (Lactobacillus reuteri or Lactobacillus casei) were formed in an agar medium and preculture was performed under the culture conditions for 48 hours. Next, cultured colonies in the preculture were transferred to 1L of a fresh BHI liquid medium and main culture was performed to a subconfluent state (OD600 = 2.5 to 3.5) under the culture conditions for 24 hours. The culture solution in the main culture was subjected to centrifugation (6,000 xg, 15 minutes, 4°C) to recover the supernatant. The supernatant was filtered on a filter with a pore diameter of 0.22 µm to recover the filtrate. Then, the filtrate was subjected to ultracentrifugation (100,000 g, 70 minutes, 4°C) to recover the precipitation fraction (pellets). In 10 mL of saline, 1 g of pellets were suspended and the obtained suspension liquid was recovered as a membrane vesicle (MV) sample. The MV sample was subjected to a nanoparticle analysis system (trade name: NanoSight LM10, laser 405 nm, Malvern Panalytical Ltd.) and the particle diameter distribution of membrane vesicles contained in the MV sample was checked. NTA3.4 (Malvern Panalytical Ltd.) was used as an analysis software.

The results of particle diameter distribution of the MV sample derived from Lactobacillus reuteri (1 g of pellets/10 mL) are shown below.
Membrane vesicle concentration: 9.35 x 10¹⁰ particles/mL
Mean: 121.4 nm,
Mode: 93 nm,
SD: 52.1 nm, and
Median diameter: D10 71.4 nm,
   D50 104.7 nm, and
   D90 208.8 nm.

The results of particle diameter distribution of the MV sample derived from Lactobacillus casei (1 g of pellets/10 mL) are shown below.
Membrane vesicle concentration: 1.5 x 10¹⁰ particles/mL,
Mean: 134.3 nm,
Mode: 98.6 nm,
SD: 49.5 nm, and
Median diameter: D10 92.3 nm,
   D50 120.0 nm, and
   D90 210.2 nm.

### [Example 2]

The ability of inhibiting the growth of a periodontal pathogen of MV derived from Lactobacillus reuteri and MV derived from Lactobacillus casein was evaluated.

### (1) Fusobacterium nucleatum

Fusobacterium nucleatum KWIK-STIK strain (ATCC 25586) was used as the periodontal pathogen. The Lactobacillus reuteri MV sample and the Lactobacillus casei MV sample prepared in Example 1 described above were used as the membrane vesicles. The MV samples were diluted with sterile water as needed.

The Lactobacillus reuteri MV sample or the Lactobacillus casei MV sample was added to a sterile ABHK agar medium in a liquid state to predetermined concentrations (0%, 1%, 5%) and the ABHK agar medium was solidified on a plate. One platinum loopful of bacteria of the genus Fusobacterium were picked and suspended in 8 mL of a ABHK liquid medium not containing agar. Then, the suspended bacteria were seeded in the plate and cultured at 37°C under anaerobic conditions for 24 hours. The bacteria of the genus Fusobacterium were visually checked on the plate after culturing and the CFU (colony forming unit) was counted.

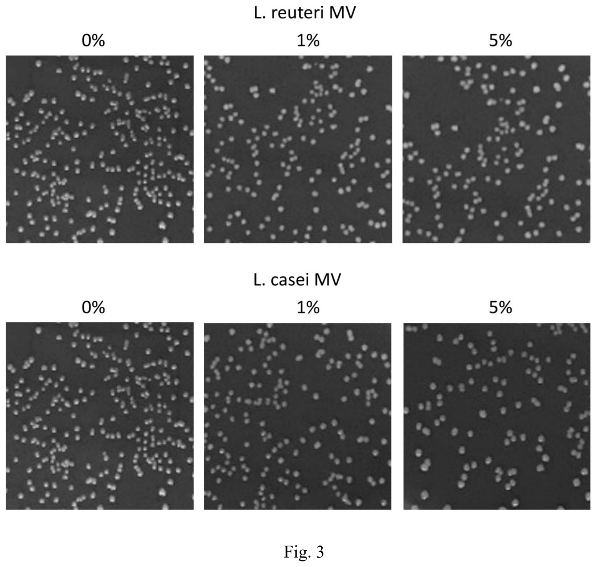

The photographs of the plates with bacteria of the genus Fusobacterium are shown in Fig. 3 and the results of CFU are shown in Table 1. Fig. 3 is the photographs showing the culture state of bacteria of the genus Fusobacterium in the presence of MV, and the upper row shows the results in the presence of Lactobacillus reuteri MV and the lower row shows the results in the presence of Lactobacillus casei. Both the upper and lower rows show the results of MV concentrations of 0%, 1% and 5% from the left.

**[Table 1]**

| *Fusobacterium nucleatum* | | | |
|---|---|---|---|
| MV | CFU | | |
| | Added concentration of MV | | |
| | 0% | 1% | 5% |
| Lactobacillus reuteri MV | 537 | 371 | 308 |
| Lactobacillus casei MV | 537 | 421 | 307 |

As shown in Fig. 3 and Table 1, using either of the Lactobacillus reuteri MV and the Lactobacillus casei MV, the number of viable cells of bacteria of the genus Fusobacterium was reduced by adding MV and moreover the number of viable cells was reduced depending on the concentration of MV. These results could verify that the growth of bacteria of the genus Fusobacterium could be inhibited by the Lactobacillus reuteri MV and the Lactobacillus casei MV.

### (2) Aggregatibacter actinomycetemcomitans

Aggregatibacter actinomycetemcomitans (standard strain LYFO DISC, ATCC 29522) and Porphyromonas gingivalis (standard strain KWIK-STIK, ATCC 33277) were used as the periodontal pathogen. The Lactobacillus reuteri MV sample and Lactobacillus casei MV sample prepared in Example 1 described above were used as the membrane vesicles. The MV samples were diluted with sterile water as needed.

The periodontal pathogen was cultured in the presence of the Lactobacillus reuteri MV sample or the Lactobacillus casei MV sample in the same manner as in (1) above except that A. actinomycetemcomitans and P. gingivalis each were used as the periodontal pathogen, and the culture state was checked.

The results of CFU of A. actinomycetemcomitans are shown in Table 2 and the results of CFU of P. gingivalis are shown in Table 3. As shown in Table 2 and Table 3, using either of the Lactobacillus reuteri MV and the Lactobacillus casei MV, the number of viable cells of the periodontal pathogen was reduced by adding MV and moreover the number of viable cells was reduced depending on the concentration of MV. These results could verify that the growth of not only bacteria of the genus Fusobacterium but also other periodontal pathogens could be inhibited by the Lactobacillus reuteri MV and the Lactobacillus casei MV.

**[Table 2]**

| *A. actinomycetemcomitans* | | | |
|---|---|---|---|
| | MV | CFU | |
| | | Added concentration of MV | |
| | | 0% | 5% |
| 1 | Lactobacillus reuteri MV | 227 | 124 |
| | Lactobacillus Casei MV | 231 | 156 |
| 2 | Lactobacillus reuteri MV | 157 | 131 |
| | Lactobacillus casei MV | 220 | 199 |

**[Table 3]**

| *P. gingivalis* | | | |
|---|---|---|---|
| | MV | CFU | |
| | | Added concentration of MV | |
| | | 0% | 5% |
| 1 | Lactobacillus reuteri MV | 359 | 228 |
| | Lactobacillus casei MV | 307 | 244 |
| 2 | Lactobacillus reuteri MV | 443 | 303 |
| | Lactobacillus casei MV | 502 | 387 |

### [Example 3]

The improvement in the ability of inhibiting the growth by isolating membrane vesicles was checked.

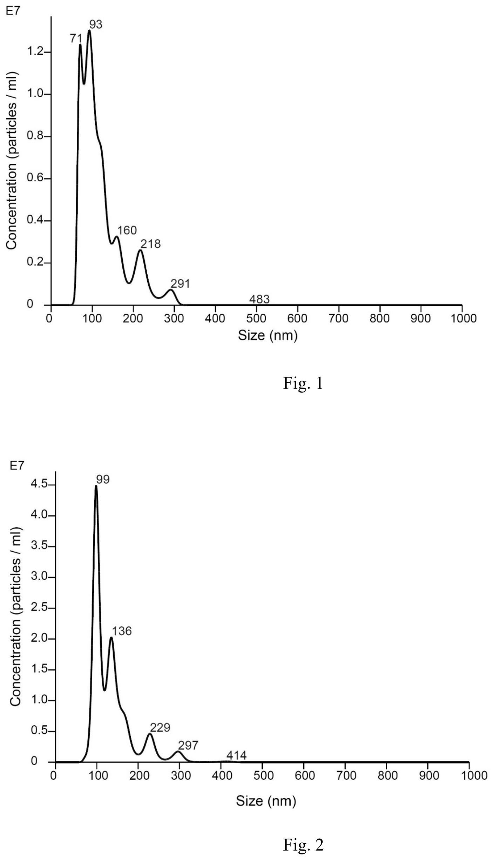

Lactobacillus reuteri was cultured, filtered and ultracentrifugated in the same manner as in Example 1 described above. In the course thereof, the culture solution of the main culture was sampled at the completion of the main culture of Lactobacillus reuteri (sample 1: original culture solution sample). In addition, the culture solution was separated into the precipitation fraction containing MV and supernatant by the ultracentrifugation and the supernatant was sampled (sample 2: MV removed sample). The precipitation fraction containing MV was suspended in saline in the same amount as of the medium used for the main culture to obtain a membrane vesicle (MV) sample (sample 3). The particle diameter distribution of the MV sample prepared from the precipitation fraction was the same as in Fig. 1 in Example 1 described above.

Then, bacteria of the genus Fusobacterium were cultured in the presence of 1 mL of each sample in the same manner as in Example 2 (1) described above, and the culture state was checked. The culture state of untreated control obtained by adding 1 mL of saline in place of a sample was checked in the same manner. The results of CFU of bacteria of the genus Fusobacterium are shown in Table 4. In addition, the CFU of untreated control is considered "1" and the relative values of CFU when using each sample are shown altogether. A smaller relative value shows that the growth is more inhibited.

**[Table 4]**

| Sample | CFU | Relative value |
|---|---|---|
| Control Untreated | 224 | 1 |
| 1: Original culture solution | 191 | 0.85 |
| 3: MV | 122 | 0.54 |

As shown in Table 4, it could be verified that the original culture solution containing the Lactobacillus reuteri themselves (sample 1) also inhibited the growth by comparison with untreated control; however, the MV sample recovered from the original culture solution (sample 3) could significantly inhibit the growth of bacteria of the genus Fusobacterium. From the results, it was found that growth could be more efficiently inhibited by not using a culture solution containing bacterial cells themselves but separating secreted membrane vesicles from bacterial cells. Because the volume of the sample 3 was the same as the volume of the culture solution used to recover EV, it can be said that the effect is obtained not by the concentration of EV but by isolated EV.

The invention of this application has been described above with reference to the embodiment. It should be noted, however, that the invention of this application is not limited to the above embodiment. Various modifications which those skilled in the art can understand can be made to the structure and details of the invention of this application without departing from the scope of the invention of this application.

This application claims priority based on Japanese patent application No. 2023-68906 filed on April 19, 2023, the entire disclosure of which is incorporated herein.

### [INDUSTRIAL APPLICABILITY]

According to the present invention, the growth of periodontal pathogens can be inhibited and thus, for example, diseases caused by periodontal pathogens can be improved by inhibiting the growth thereof.

## Claims

1. A periodontal pathogen growth inhibitor, **characterized by** comprising at least one of membrane vesicles of Lactobacillus reuteri and membrane vesicles of Lactobacillus casei.

2. The growth inhibitor according to claim 1, wherein the periodontal pathogen is at least one selected from a group consisting of bacteria of genus Fusobacterium, bacteria of genus Porphyromonas and bacteria of genus Tannerella.

3. The growth inhibitor according to claim 2, wherein the bacteria of the genus Fusobacterium are Fusobacterium nucleatum.

4. The growth inhibitor according to claim 2, wherein the bacteria of the genus Porphyromonas are Porphyromonas gingivalis.

5. The growth inhibitor according to claim 2, wherein the bacteria of the genus Tannerella are Tannerella forsythensis.

6. The growth inhibitor according to any one of claims 1 to 5, wherein the membrane vesicles are membrane vesicles isolated from cultures of Lactobacillus reuteri.

7. The growth inhibitor according to any one of claims 1 to 5, wherein the membrane vesicles are membrane vesicles isolated from cultures of Lactobacillus casei.

8. A method for inhibiting growth of a periodontal pathogen, **characterized in that** the periodontal pathogen growth inhibitor according to any one of claims 1 to 7 and a periodontal pathogen are allowed to coexist.

9. The growth inhibition method according to claim 8, wherein the coexistence is coexistence in vivo or in vitro.

10. The growth inhibition method according to claim 8 or 9, wherein the coexistence is coexistence in a living body of a human or a non-human animal.

11. A drug for a periodontal pathogen-related disease, **characterized by** comprising the periodontal pathogen growth inhibitor according to any one of claims 1 to 7 as an active ingredient.

12. The drug for a periodontal pathogen-related disease according to claim 11, further comprising an excipient.

13. The drug for a periodontal pathogen-related disease according to claim 11 or 12, wherein the periodontal pathogen-related disease is myocardial infarction, cerebral infarction, an oral disease or a gastrointestinal disease.

14. The drug for a periodontal pathogen-related disease according to claim 13, wherein the oral disease is periodontal disease or tooth decay.

15. The drug for a periodontal pathogen-related disease according to claim 13, wherein the gastrointestinal disease is large bowel disease.

16. The drug for a periodontal pathogen-related disease according to claim 15, wherein the large bowel disease is colitis or colorectal cancer.

17. Membrane vesicles of Lactobacillus reuteri or membrane vesicles of Lactobacillus casei for use in inhibition of growth of a periodontal pathogen or a drug for a periodontal pathogen-related disease.

18. Membrane vesicles of Lactobacillus reuteri or membrane vesicles of Lactobacillus casei for use in production of a periodontal pathogen growth inhibitor or a drug for a periodontal pathogen-related disease.
